# EUROPEAN PATENT APPLICATION

(11) **EP 4 699 659 A2**
(43) Date of publication of application: **25.02.2026**
(21) Application number: 25227462.6
(22) Date of filing: 24.04.2024
(51) Int. Cl.: A61P 25/18

(54) **CRYSTALLINE FORMS OF EVENAMIDE**

(30) Priority: 28.04.2023 EP 23170709
(62) Divisional of application: 24720258.3
(71) Applicant: Newron Pharmaceuticals S.p.A., 20091 Bresso (MI) (IT)
(72) Inventor: IMBODEN, Christoph, 4114 Hofstetten, Solothurn (CH); CASTELLIN, Andrea, 35035 Mestrino (PD) (IT)
(74) Representative: Bianchetti & Minoja with Trevisan & Cuonzo IPS SRL

(57) **Abstract**

The present invention relates to novel crystalline forms of evenamide salts, in particular forms I and II, their use a process for their preparation, and a pharmaceutical composition containing said novel crystalline forms.

## Description

### TECHNICAL FIELD OF INVENTION

The present invention relates to crystalline forms of evenamide salts, preferably the hydrochloride salt, in particular form I and form II, their use, a process for their preparation, and a pharmaceutical composition containing said novel crystalline forms.

### BACKGROUND OF THE INVENTION

Evenamide, 2-[2-(3-butoxyphenyl)-ethylamino]-N,N-dimethylacetamide, is a new chemical entity, orally available, that specifically targets voltage-gated sodium channels (VGSCs), having the following chemical formula (I):

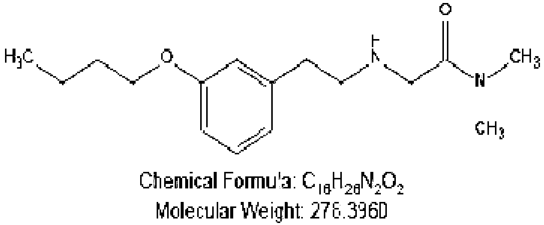

Evenamide modulates sustained repetitive firing, without inducing impairment of normal excitability. It normalizes glutamate release induced by aberrant sodium channel activity and is under clinical investigation as add-on therapy for the treatment of schizophrenia.

Evenamide has demonstrated potential benefits in a battery of animal models predictive of efficacy in schizophrenia and other psychiatric diseases. In phase 2 studies in schizophrenic patients evenamide has provided preliminary evidence of efficacy, tolerability and safety.

WO 2008/151702 discloses in the examples the synthesis of 2-[2-(3-butoxyphenyl)-ethylamino]-N,N-dimethylacetamide and its hydrochloride salt as summarized in the following Scheme A:

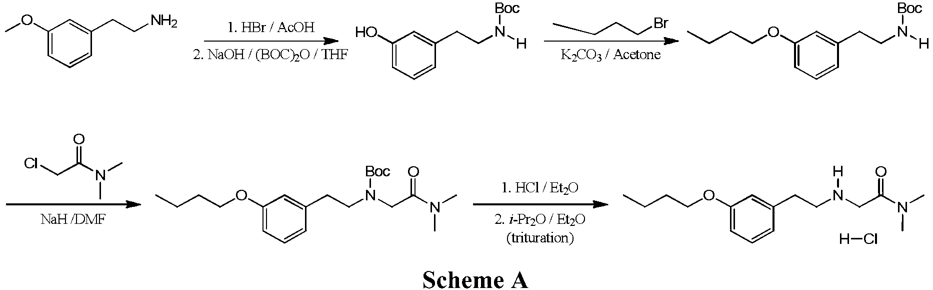

WO 2020/212227 discloses a process for the preparation of evenamide, its hydrochloride salt and deuterated derivatives thereof.

WO 2020/212352 discloses another process for the preparation of evenamide, its hydrochloride salt and deuterated derivatives thereof.

IN201911052571 discloses a process of preparing an amorphous form of evenamide or pharmaceutically acceptable salts thereof.

Crystalline forms of evenamide have never been disclosed and characterized.

There is consequently a need to provide crystalline forms of evenamide that are stable, not hygroscopic, particularly soluble in water and biological fluids, with optimum characteristics for formulation in pharmaceutical technology, and also advantageously usable as intermediates for the preparation of crystalline forms of evenamide, in particular in salt form.

### SUMMARY OF THE INVENTION

The present invention relates to crystalline forms of evenamide salts, preferably the hydrochloride salt and specifically crystalline forms of evenamide hydrochloride salt hereinafter defined as crystalline forms I and II and crystalline form III is also disclosed, a process for their preparation, and a pharmaceutical composition containing at least one of said crystalline forms.

### BRIEF DESCRIPTION OF FIGURES AND ANALYSIS METHODS

Figure 1: XRPD spectrum of evenamide hydrochloride in crystalline form I.
Figure 2: DSC curve of evenamide hydrochloride in crystalline form I and II.
Figure 3: DSC and TGA curves of evenamide hydrochloride in crystalline form I and form II.
Figure 4: HSM images of evenamide hydrochloride in crystalline form I and II, A: obtained at T= 25°C; B: obtained at T= 80°C; C: obtained at T= 110°C; D: obtained at T= 140°C.
Figure 5: comparison of XRPD patterns at 80°C of evenamide hydrochloride in crystalline
form I and at 110°C of evenamide hydrochloride in crystalline form II.
Figure 6: comparison of XRPD patterns of evenamide hydrochloride in crystalline form I at room temperature before and after heating/cooling treatment.
Figure 7: XRPD spectrum of evenamide hydrochloride in crystalline form III.

The crystalline forms of evenamide hydrochloride salt were characterized by X-ray powder diffraction (XRPD).

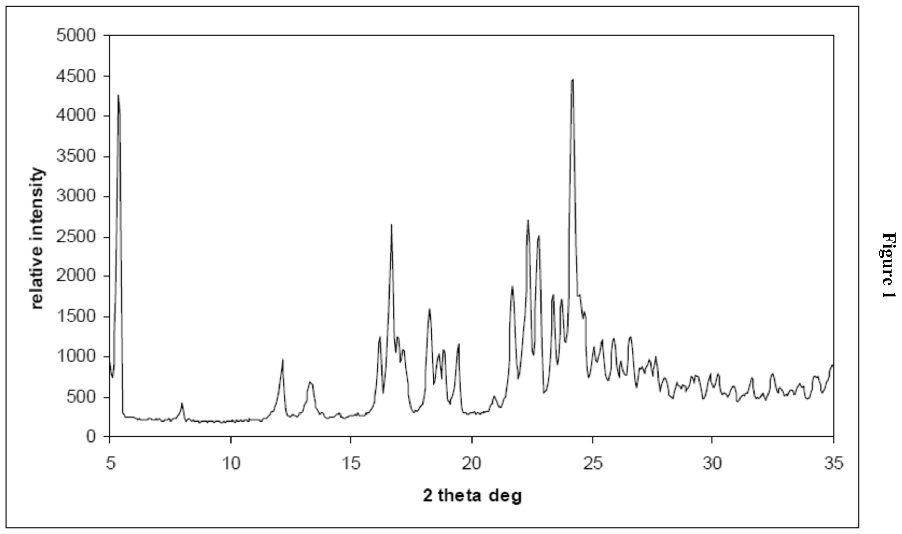

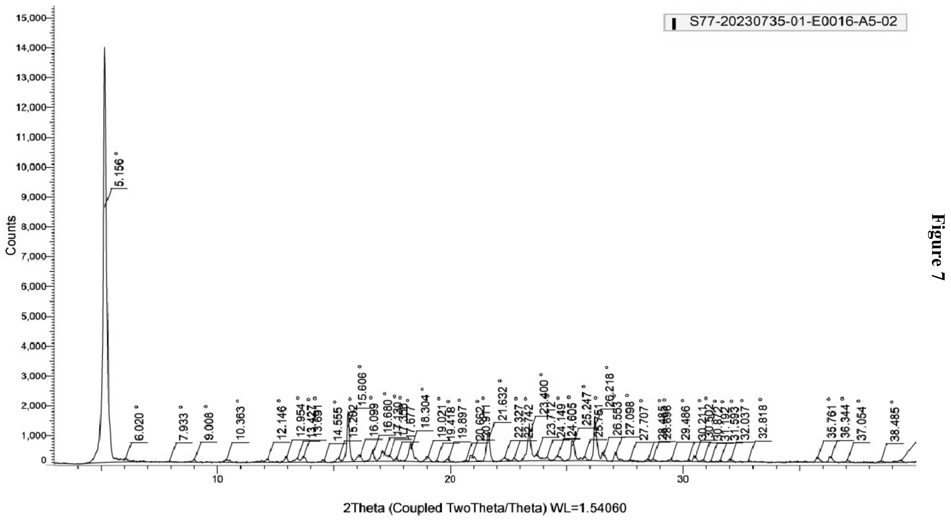

The X-ray powder diffraction (XRPD) pattern of Figure 1 and 7 was collected with a Rigaku Miniflex diffractometer using Cu-Kα radiation with wavelength λ = 1.54 Å generated with 30 kV and 15 mA. Powder samples gently ground in a mortar were packed in aluminium sample holders and XRPD patterns were recorded using the step scan mode at a scan speed of 1.0° 20 min⁻¹ in the 2θ angle of range 5 to 35°.

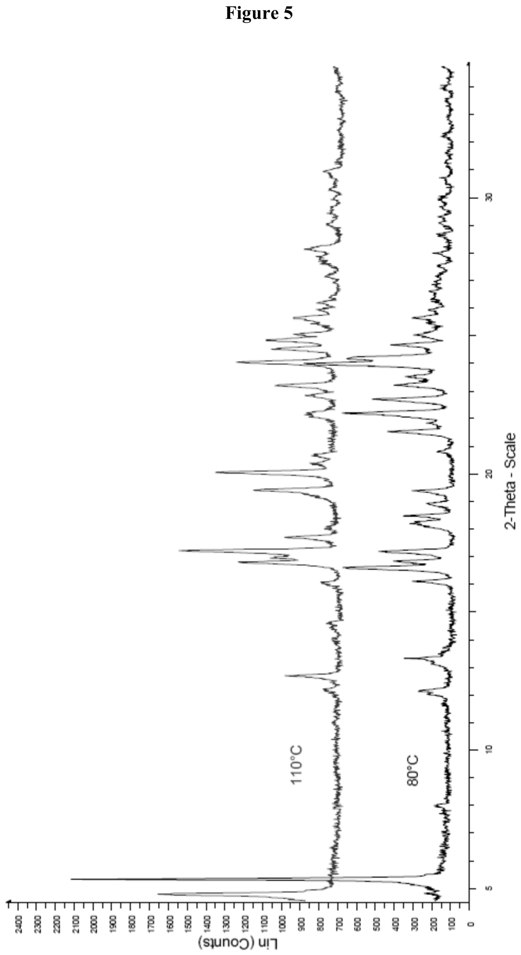

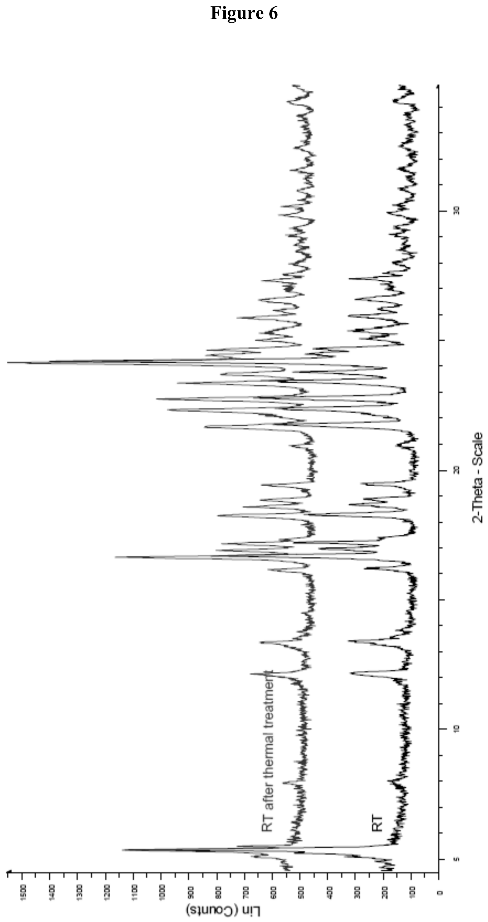

XRPD patterns of Figure 5 and Figure 6 have been obtained by measurements taken on the samples at 80°C and 110°C, after previous gentle grinding in an agata mortar in a TTK Anton Paar High Temperature device using a Bruker BD5005 diffractometer with Cu-Kα radiation with wavelength λ = 1.54 Å, Ni filter, and position sensitive detector (PSD) at the following conditions: 40 k V, 30 mA, stepwidth 0.0 15° (2 θ), steptime 0.30 s/step, in the angular range 4.5<2 0 <35.

The DSC patterns were obtained with a differential scanning calorimeter, using a DSC2010 TA Instruments calorimeter under nitrogen purging of 70 mL min⁻¹. Runs were performed on 2-3 mg samples in non-hermetically sealed aluminum pans at 10 K·min⁻¹ scanning rate, from 25 to 300°C.

TGA curves were recorded on a TG2050 TA Instruments thermogravimetric analyzer at a heating rate of 10 K·min⁻¹ with a nitrogen purge of 90 mL/min.

HSM images were obtained by examining powder samples by a Hot Stage Microscope apparatus consisting of a Mettler FP82HT cell, a Mettler FP 82HT control unit and an optical microscope (Reichert Microstar I).

Some crystals were suspended in silicon oil and placed on a microscope slide, covered with a cover glass and heated at a scanning rate of 5 K·min⁻¹, from 25°C to 160°C. Photomicrograph were taken at predetermined temperatures.

### DETAILED DESCRIPTION OF THE INVENTION

The present invention relates to solid crystalline forms of salts of 2-[2-(3-butoxyphenyl)-ethylamino]-N,N-dimethylacetamide of formula (I), also known as evenamide, preferably the hydrochloride salt, selected from:
- evenamide in crystalline form, defined herein as form I, having an XRPD spectrum, for example as shown in Figure 1, wherein the characteristic peaks are found at about 12.1; 13.4; 16.2; 16.7; 18.2; 18.7; 19.4; 21.7; 22.4; 22.8; 23.3; 23.7; 24.2; 24.4; 25.4; 26.0; 26.6 ± 0.2° in 20;
- evenamide in crystalline form, defined herein as form II, having an XRPD spectrum, for example as shown in Figure 5 (at 110°C), wherein the characteristic peaks are found at about 12.7; 17.7; 20.0; 20.4; 20.7 ± 0.2° in 2θ.

The invention also discloses evenamide hydrochloride in crystalline form defined herein as form III, characterized by an XRPD spectrum as shown in Figure 7, wherein the characteristic peaks are found at about 15.6; 18.3; 21.6; 23.4; 25.2; 26.2 ± 0.2° in 2θ. Evenamide in crystalline form III was determined to be a 1,4-dioxane solvate of mono-HCl salt.

Preferably the salt, more preferably the hydrochloride salt, of evenamide is in crystalline form I.

Another object of the invention is a crystalline form of a salt of 2-[2-(3-butoxyphenyl)-ethylamino]-N,N-dimethylacetamide of formula (I) wherein the salt of evenamide is selected from salts:
- exhibiting a predominant endothermic peak at about 119°C, preferably sulfate;
- exhibiting a predominant endothermic peak at about 132.6°C, preferably maleate;
- exhibiting a predominant endothermic peak at about 105.6°C, preferably phosphate;
- exhibiting a predominant endothermic peak at about 118.3°C, preferably pyruvate;
- exhibiting a predominant endothermic peak at about 114.6°C, preferably fumarate;
- exhibiting a predominant endothermic peak at about 154.8°C, preferably mucate;
- exhibiting a predominant endothermic peak at about 140.7°C, preferably citrate;
- exhibiting a predominant endothermic peak at about 101.2°C, preferably L-malate;
- exhibiting a predominant endothermic peak at about 85.8°C, preferably succinate;
- exhibiting a predominant endothermic peak at about 91.4°C, preferably glutarate;
- exhibiting a predominant endothermic peak at about 147.9°C, preferably napadisylate;
- exhibiting a predominant endothermic peak at about 197.8°C, preferably edisylate;
- exhibiting a predominant endothermic peak at about 120.0°C, preferably esylate;
- exhibiting a predominant endothermic peak at about 161.8°C, preferably tosylate;
- exhibiting a predominant endothermic peak at about 139.5°C, preferably mesylate;
- exhibiting a predominant endothermic peak at about 184.5°C, preferably napsylate;
- exhibiting a predominant endothermic peak at about 107.0°C, preferably besilate;
- exhibiting a predominant endothermic peak at about 198.3°C, preferably oxalate;
- exhibiting a predominant endothermic peak at about 96.6°C, preferably malonate;
- exhibiting a predominant endothermic peak at about 150.1°C, preferably saccharinate;
- exhibiting a predominant endothermic peak at about 129.0°C, preferably camsylate; and - exhibiting a predominant endothermic peak at about 105.0°C, preferably mandelate;
   more preferably the salt is selected from the group comprising maleate, mucate, citrate, edisylate, mesylate, napsylate, oxalate, saccharinate and camsylate; even more preferably from mucate, citrate, edisylate, mesylate, napsylate and saccharinate.

In a further preferred embodiment, the salt is selected from the group listed in Table 1:

**Table 1**

| **No.** | **Salt Form** | **Form Identity** | **Crystallinity by XRPD** | **Endo. in DSC (peak, °C)** |
|---|---|---|---|---|
| 1 | Hydrochloride | | High | |
| 2 | Sulfate Type B | Hydrate (mono salt) | High | 64.9, 119.1 melt |
| 3 | Sulfate Type C | Hydrate (hemi salt) | High | 66.8, 86.0, 132.8 melt |
| 4 | Maleate Type A | Anhydrate (mono salt) | High | 112.3, 132.6 melt |
| 5 | Maleate Type B | Anhydrate (mono salt) | High | Obtained by heating maleate Type A to 120 °C |
| 6 | Phosphate Type A | Hydrate (mono salt) | High | 105.6 melt |
| 7 | Pyruvate Type A | Anhydrate (mono salt) | High | 78.7, 118.3 melt |
| 8 | Pyruvate Type B | Anhydrate (mono salt) | High | Obtained by heating pyruvate Type A to 80 °C |
| 9 | Fumarate Type A | Anhydrate (mono salt) | High | 114.6 melt |
| 10 | Fumarate Type B | Hydrate (hemi salt) | High | 104.4, 107.9, 114.4 melt |
| 11 | Fumarate Type C | Anhydrate (hemi salt) | High | 110.1, 114.7 melt |
| 12 | Mucate Type A | Anhydrate (mono salt) | High | 154.8 melt |
| 13 | Citrate Type A | Anhydrate (mono salt) | High | 140.7 melt |
| 14 | L-malate Type A | Anhydrate (mono salt) | High | 101.2 melt |
| 15 | Succinate Type A | Anhydrate (mono salt) | High | 85.8 melt |
| 16 | Succinate Type B | Anhydrate (mono salt) | High | 85.6 melt |
| 17 | Glutarate Type A | Anhydrate (mono salt) | High | 91.4 melt |
| 18 | Napadisylate Type A | Hydrate (mono salt) | High | 51.1, 76.4, 116.7 melt |
| 19 | Napadisylate Type B | Hydrate (hemi salt) | High | 31.8, 72.3, 147.9 melt |
| 20 | Edisylate Type A | Anhydrate (hemi salt) | High | 115.0, 148.0, 197.8 melt |
| 21 | Edisylate Type B | Anhydrate (hemi salt) | High | 148.3, 200.3 melt |
| 22 | Edisylate Type C | Anhydrate (hemi salt) | High | Obtained by heating edisylate Type B to 150 °C |
| 23 | Esylate Type A | Anhydrate (mono salt) | High | 120.0 melt |
| 24 | Tosylate Type A | Hydrate (mono salt) | High | 77.7, 161.8 melt |
| 25 | Tosylate Type B | Hydrate (mono salt) | High | 66.3, 161.8 melt |
| 26 | Mesylate Type A | Anhydrate (mono salt) | High | 139.5 melt |
| 27 | Napsylate Type A | Anhydrate (mono salt) | High | 184.5 melt |
| 28 | Besylate Type A | Hydrate (mono salt) | High | 68.3, 107.0 melt |
| 29 | Oxalate Type A | Anhydrate (mono salt) | High | 123.3, 198.3 melt w/decomp. |
| 30 | Oxalate Type B | Anhydrate (mono salt) | High | Obtained by heating oxalate Type A to 125 °C |
| 31 | Malonate Type A | Anhydrate (mono salt) | High | 96.6 melt |
| 32 | Saccharinate Type A | Anhydrate (mono salt) | High | 150.1 melt |
| 33 | Camsylate Type A | Anhydrate (mono salt) | High | 129.0 melt |
| 34 | Mandelate Type A | Anhydrate (mono salt) | High | 105.0 melt |

| | | | | |
|---|---|---|---|---|
| more preferably, the salt is selected from the group listed in Table 2: | | | | |

**Table 2**

| **No.** | **Salt Form** | **Form Identity** | **Crystallinity by XRPD** | **Endo. in DSC (peak, °C)** |
|---|---|---|---|---|
| 1 | Hydrochloride | | High | |
| 4 | Maleate Type A | Anhydrate (mono salt) | High | 112.3, 132.6 melt |
| 12 | Mucate Type A | Anhydrate (mono salt) | High | 154.8 melt |
| 13 | Citrate Type A | Anhydrate (mono salt) | High | 140.7 melt |
| 20 | Edisylate Type A | Anhydrate (hemi salt) | High | 115.0, 148.0, 197.8 melt |
| 21 | Edisylate Type B | Anhydrate (hemi salt) | High | 148.3, 200.3 melt |
| 26 | Mesylate Type A | Anhydrate (mono salt) | High | 139.5 melt |
| 27 | Napsylate Type A | Anhydrate (mono salt) | High | 184.5 melt |
| 29 | Oxalate Type A | Anhydrate (mono salt) | High | 123.3, 198.3 melt |
| 32 | Saccharinate Type A | Anhydrate (mono salt) | High | 150.1 melt |
| 33 | Camsylate Type A | Anhydrate (mono salt) | High | 129.0 melt |

| | | | | |
|---|---|---|---|---|
| even more preferably, the salt is selected from the group listed in Table 3: | | | | |

**Table 3**

| **No.** | **Salt Form** | **Form Identity** | **Crystallinity by XRPD** | **Endo. in DSC (peak, °C)** |
|---|---|---|---|---|
| 1 | Hydrochloride | | High | |
| 12 | Mucate Type A | Anhydrate (mono salt) | High | 154.8 melt |
| 13 | Citrate Type A | Anhydrate (mono salt) | High | 140.7 melt |
| 20 | Edisylate Type A | Anhydrate (hemi salt) | High | 115.0, 148.0, 197.8 melt |
| 21 | Edisylate Type B | Anhydrate (hemi salt) | High | 148.3, 200.3 melt |
| 26 | Mesylate Type A | Anhydrate (mono salt) | High | 139.5 melt |
| 27 | Napsylate Type A | Anhydrate (mono salt) | High | 184.5 melt |
| 32 | Saccharinate Type A | Anhydrate (mono salt) | High | 150.1 melt |

Preferred salts according to the present invention are those salts which have a melting point equal to or higher than 120°C and are neither solvates nor hydrates.

More preferred salts according to the present invention are those salts with a melting point equal to or higher than 130°C.

Most preferred salts are those polymorphs stable at room temperature, that is from about 20 to about 25°C.

Comparative salts are reported in Table 4.

**Table 4**

| **No.** | **Salt Form** | **Form Identity** | **Crystallinity by XRPD** | **Endo. in DSC (peak, °C)** |
|---|---|---|---|---|
| 35 | Sulfate Type A | Meta-stable | High | Converted to sulfate Type A+B or B after vacuum drying at 30 °C overnight |
| 36 | Gentisate Type A | Anhydrate (mono salt) | Low | 83.1 melt |

The term "crystalline forms of evenamide" according to the present invention means solid crystalline forms of evenamide, as salts thereof, including polymorphs, tautomers, solvates.

The term "solvate" as used herein, refers to a crystal form that incorporates a solvent in the crystal structure. When the solvent is water, the solvate is referred to as a "hydrate". The solvent in a solvate may be present in either a stoichiometric or in a non-stoichiometric amount.

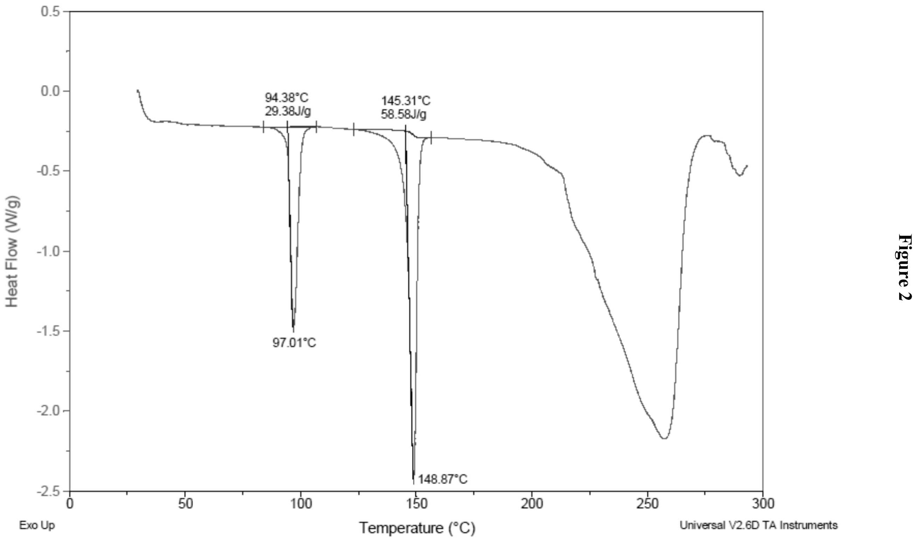

Evenamide, hydrochloride, in crystalline form I and II, as defined above, are further characterized by a DSC curve as shown in Figure 2.

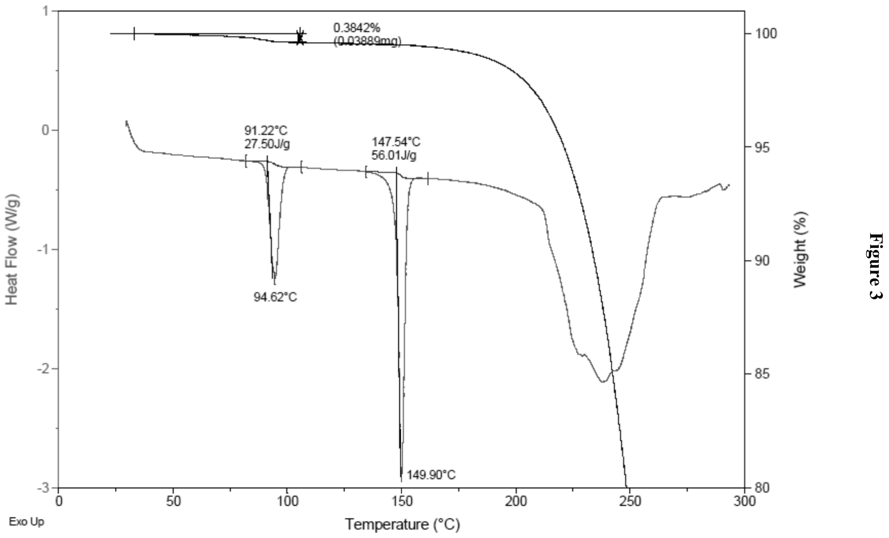

Evenamide, hydrochloride, in crystalline form I and II, as defined above, are further characterized by DSC and TGA curves as shown in Figure 3.

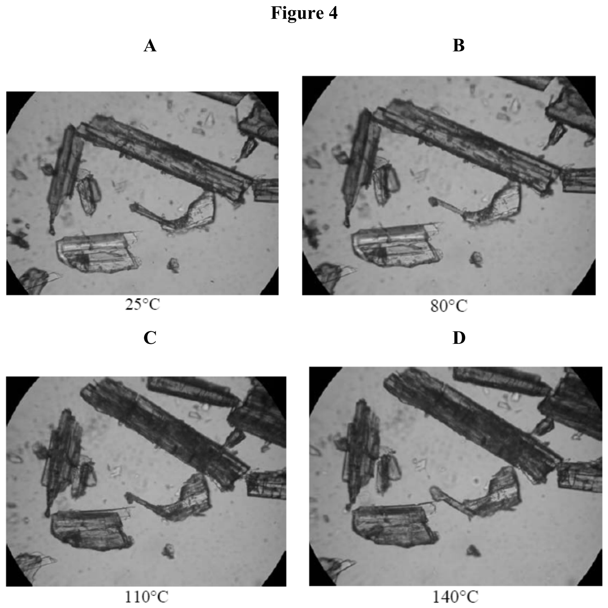

Evenamide, hydrochloride, in crystalline form I and II, as defined above, are further characterized by HSM images as shown in Figure 4.

Another object of the present invention is a process for the preparation of evenamide hydrochloride salt in crystalline form I according to the invention, comprising:
- adding a solvent to evenamide free base and hydrochloric acid in gas form, or aqueous hydrochloric acid, preferably hydrochloric acid in gas form, as a salification reagent;
- crystallizing at a temperature equal to or lower than 94°C;
- recovering evenamide hydrochloride salt in crystalline form I,
wherein the solvent is selected from the group comprising, or consisting of, acetone, water, methyl alcohol, isopropyl alcohol, methyl ethyl ketone/water, ethyl acetate, methyl isobutyl ketone, methyl isobutyl carbinol, dimethyl formamide, heptane(s), or a mixture of said solvents, preferably the solvent is acetone (Scheme 1).

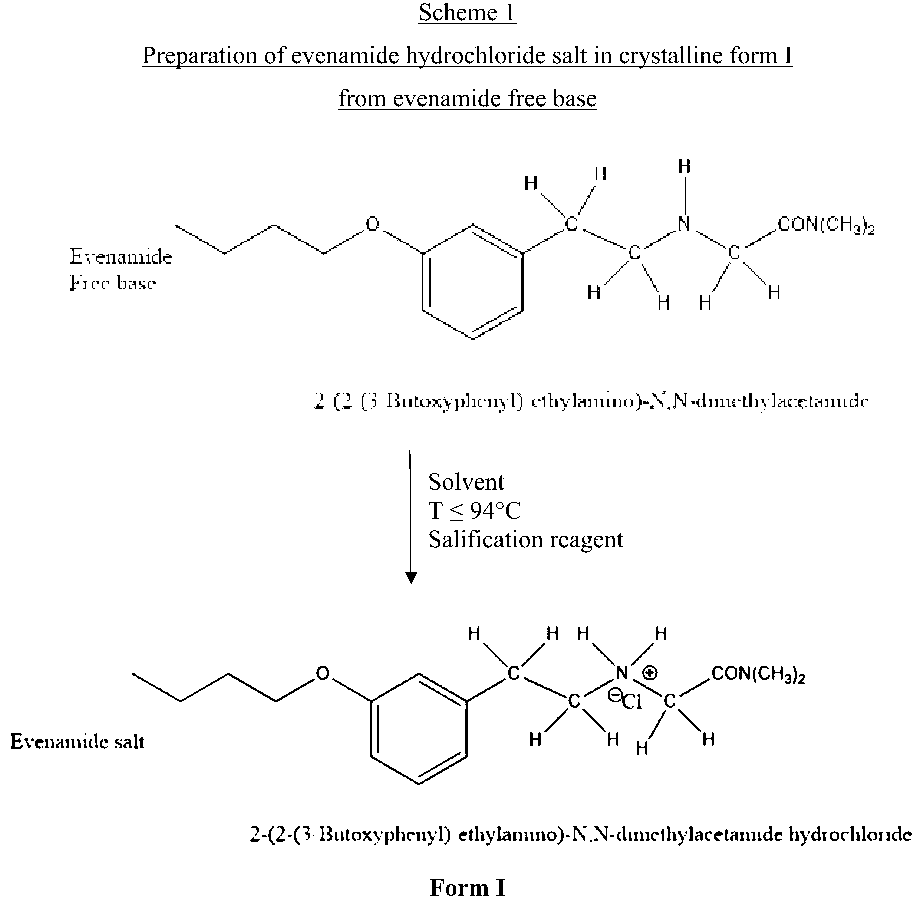

According to the present invention, the evenamide used as starting material may be in any form, crystalline or non-crystalline; anhydrous, hydrated or solvated; characterized by a high-purity of at least 99.0%, preferably 99.5%, 99.6%, 99.7%, 99.8%, more preferably 99.9%.

The molar ratio between the salification reagent and evenamide in the starting solution may range between 10:1 to 0.5:1, preferably the salification reagent and evenamide are used in a stoichiometric molar ratio.

A solution of evenamide is typically formed at a temperature ranging between -20°C and 90°C, preferably between 20°C and 30°C, more preferably at 25°C (room temperature).

The salification reagent according to the invention may be selected from hydrochloric acid in gas form, and aqueous hydrochloric acid; preferably is hydrochloric acid in gas form.

According to the invention, the solvent is selected from the group comprising, or consisting of, acetone, water, methyl alcohol, isopropyl alcohol, methyl ethyl ketone/water, ethyl acetate, methyl isobutyl ketone, methyl isobutyl carbinol, dimethyl formamide, heptane(s), or a mixture of said solvents; preferably the solvent is acetone.

In a preferred embodiment, the solvent is acetone and the salification reagent is hydrochloric acid in gas form.

Crystallization may be carried out by methods known in the art, such as by solvent evaporation or by suspension equilibration (slurry method), by cooling a solution, by precipitation, by vapor diffusion, by crystallization from the melt, by heat-induced transformations, by sublimation, by desolvation of solvates, by salting out, by pH change, preferably by solvent evaporation or by suspension equilibration (slurry method).

In the crystallization by solvent evaporation according to the invention, the solvent may be selected from the group comprising, or consisting of, methyl ethyl ketone, methyl isobutyl ketone, methyl alcohol, ethyl alcohol, water, acetone, preferably the solvent is acetone.

In the crystallization by suspension equilibration (slurry method) according to the invention, the solvent may be selected from the group comprising, or consisting of, methyl ethyl ketone, methyl isobutyl ketone, acetone, isopropyl alcohol, preferably the solvent is acetone.

The evenamide in crystalline form I, preferably the hydrochloride salt, thus obtained can be advantageously used to prepare evenamide in crystalline form II, preferably the hydrochloride salt.

A further object of the present invention is a process for the preparation of evenamide hydrochloride salt in crystalline form II according to the invention, comprising:
- adding a solvent, preferably methyl isobutyl ketone, to evenamide free base;
- heating the solution/dispersion to a temperature comprised between 99 and 147°C;
- crystallizing by adding a salification reagent;
- obtaining and optionally recovering evenamide hydrochloride salt in crystalline form II, Scheme 2.

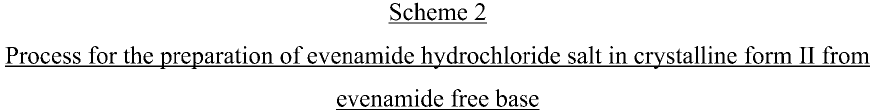

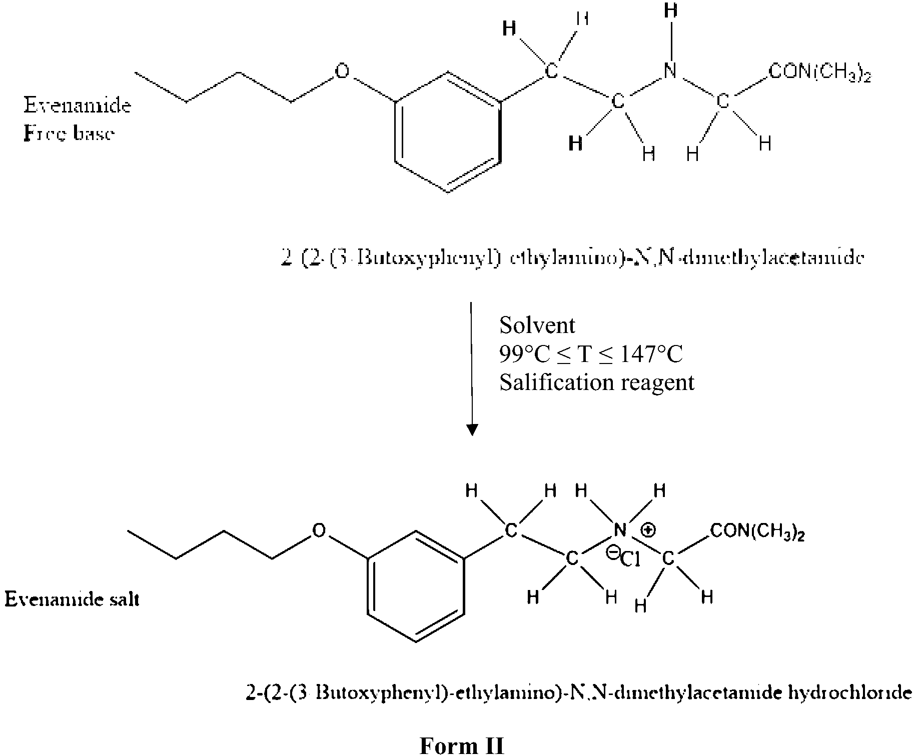

A further object of the present invention is a process for the preparation of evenamide hydrochloride salt in crystalline form II according to the invention, comprising:
- adding a solvent to evenamide free base and a salification reagent to form evenamide hydrochloride salt in crystalline form I;
- heating the solution/dispersion to a temperature comprised between 99 and 147°C;
- obtaining and optionally recovering evenamide hydrochloride salt in crystalline form II, Scheme 3.

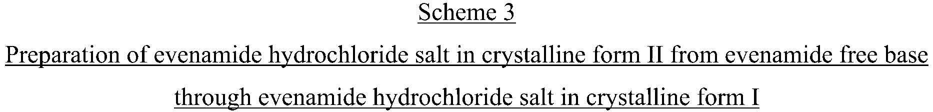

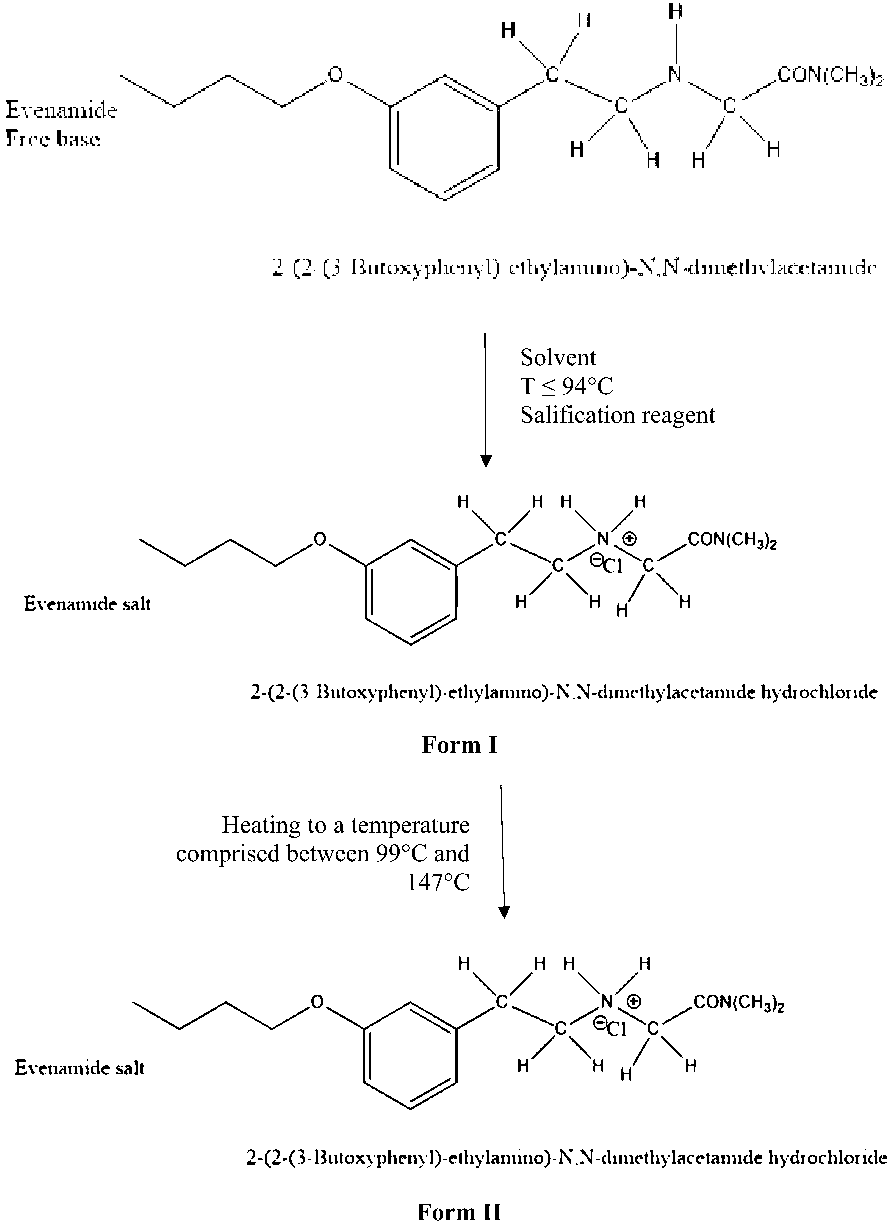

The evenamide in crystalline form II, preferably the hydrochloride salt, thus obtained can be advantageously used to prepare evenamide in crystalline form I, preferably hydrochloride salt.

A further object of the present invention is a process for the preparation of evenamide hydrochloride salt in crystalline form I according to the invention, comprising:
- adding a solvent to evenamide free base;
- heating the solution/dispersion to a temperature comprised between 99 and 147°C;
- crystallizing by adding hydrochloric acid in gas form as salification reagent;
- obtaining evenamide hydrochloride salt in crystalline form II,
- cooling down to a temperature comprised between 0 and 94°C, preferably at a temperature comprised between 20 and 30°C, more preferably of 25°C (room temperature);
- obtaining and recovering evenamide hydrochloride salt in crystalline form I, Scheme 4.

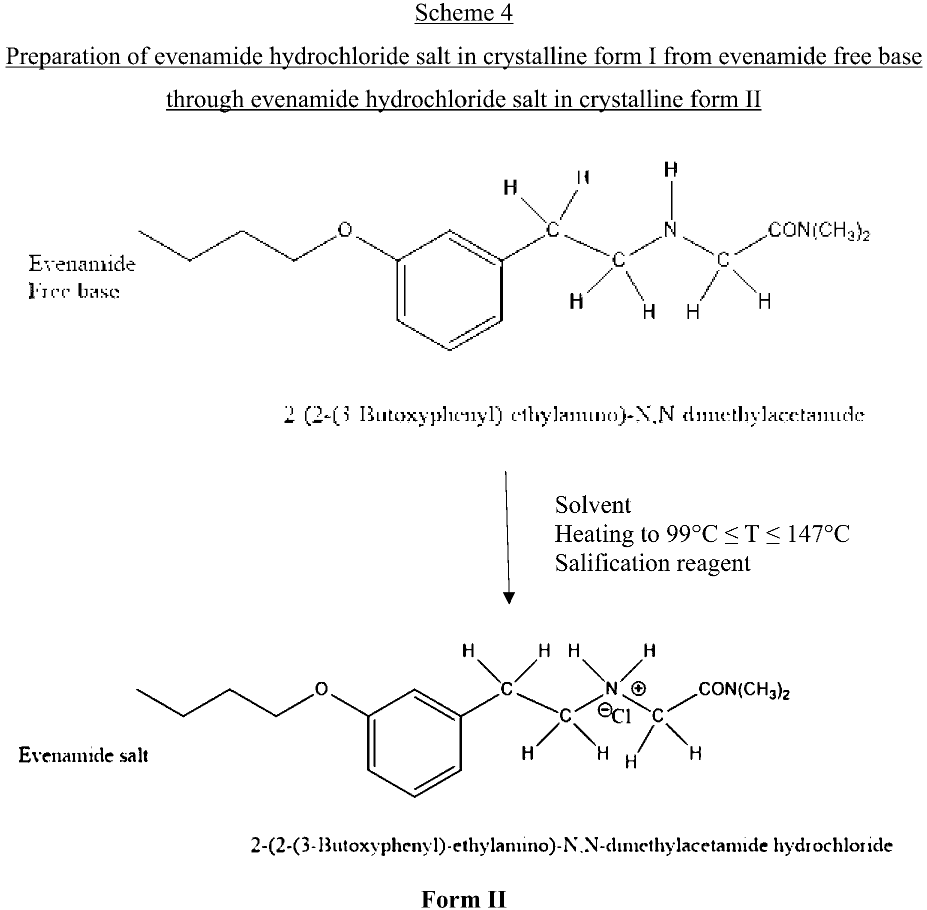

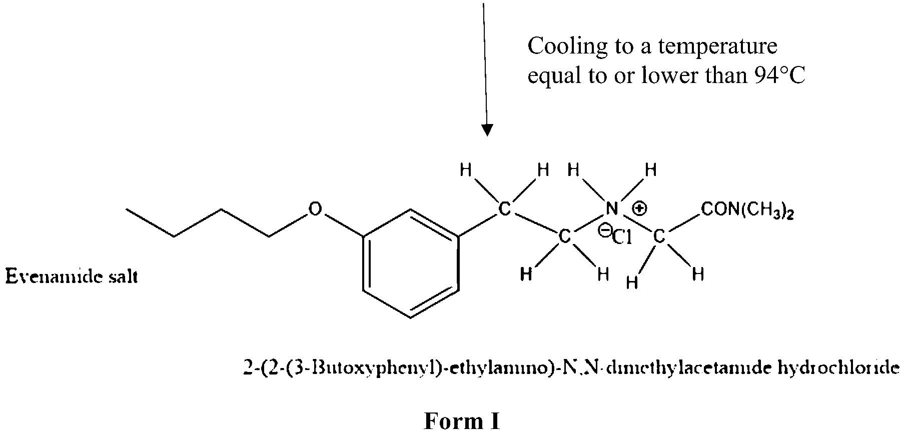

A further object of the present invention is a process for the preparation of evenamide hydrochloride salt in crystalline form I according to the invention, comprising:
- adding a solvent to evenamide free base and hydrochloric acid in gas form as salification reagent;
- heating the solution/dispersion to a temperature comprised between 99 and 147°C;
- obtaining evenamide hydrochloride salt in crystalline form II;
- cooling down to a temperature comprised between 0 and 94°C, preferably at a temperature comprised between 20 and 30°C, more preferably of 25°C (room temperature);
- obtaining and recovering evenamide hydrochloride salt in crystalline form I, Scheme 5.

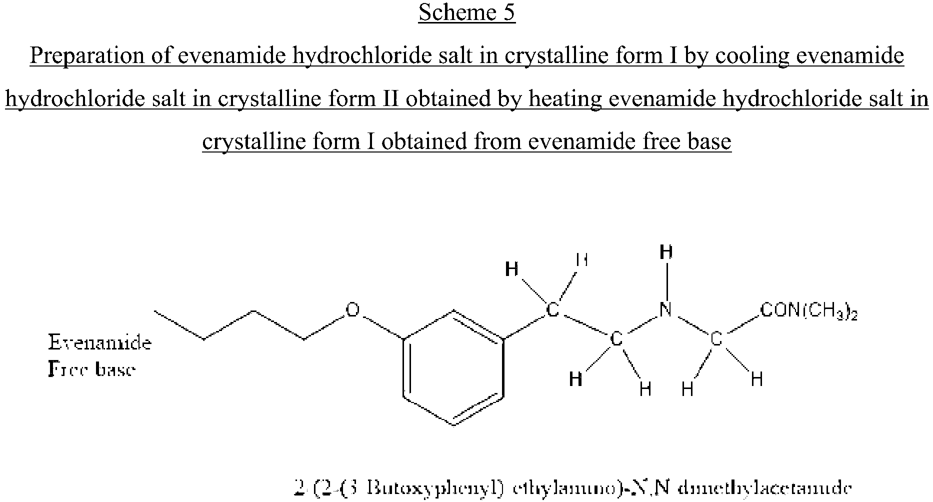

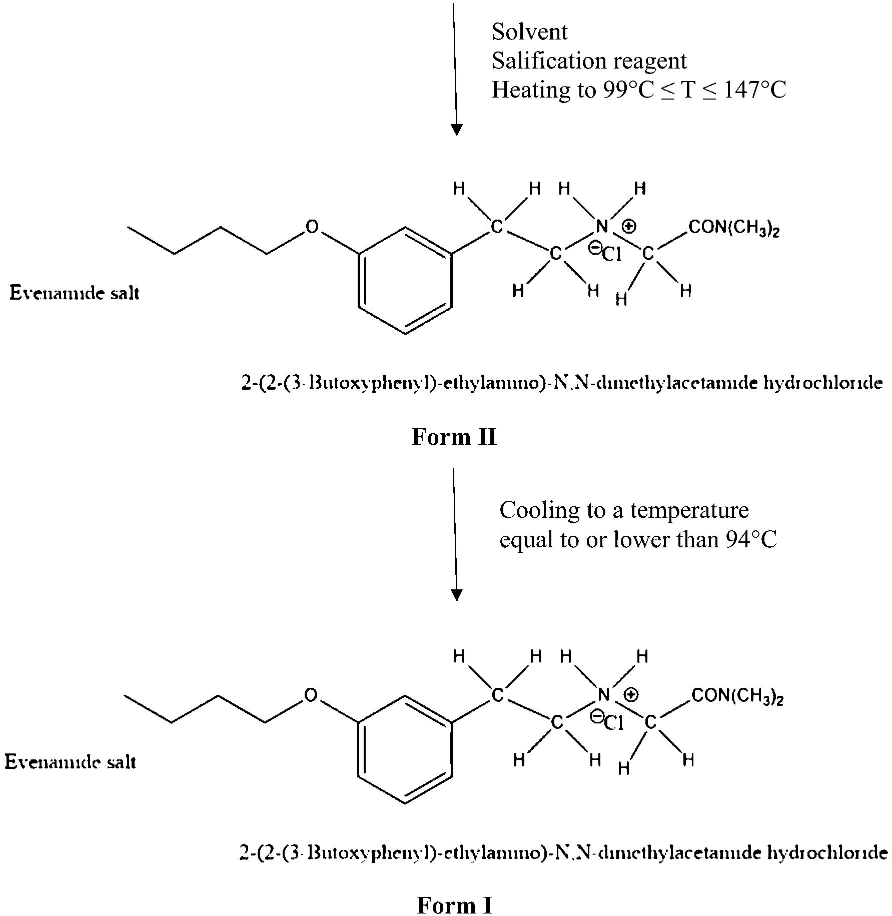

A further object of the present invention is a process for the preparation of evenamide hydrochloride salt in crystalline form II according to the invention comprising heating evenamide hydrochloride salt in crystalline form I at a temperature comprised between 99°C and 147°C and a process for the preparation of evenamide hydrochloride salt in crystalline form I according to the invention comprising cooling evenamide hydrochloride salt in crystalline form II at a temperature equal to or lower than 94°C, Scheme 6.

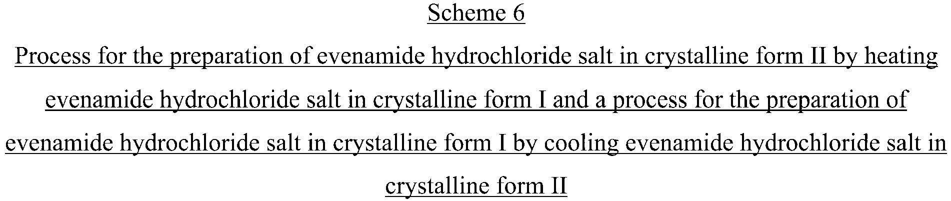

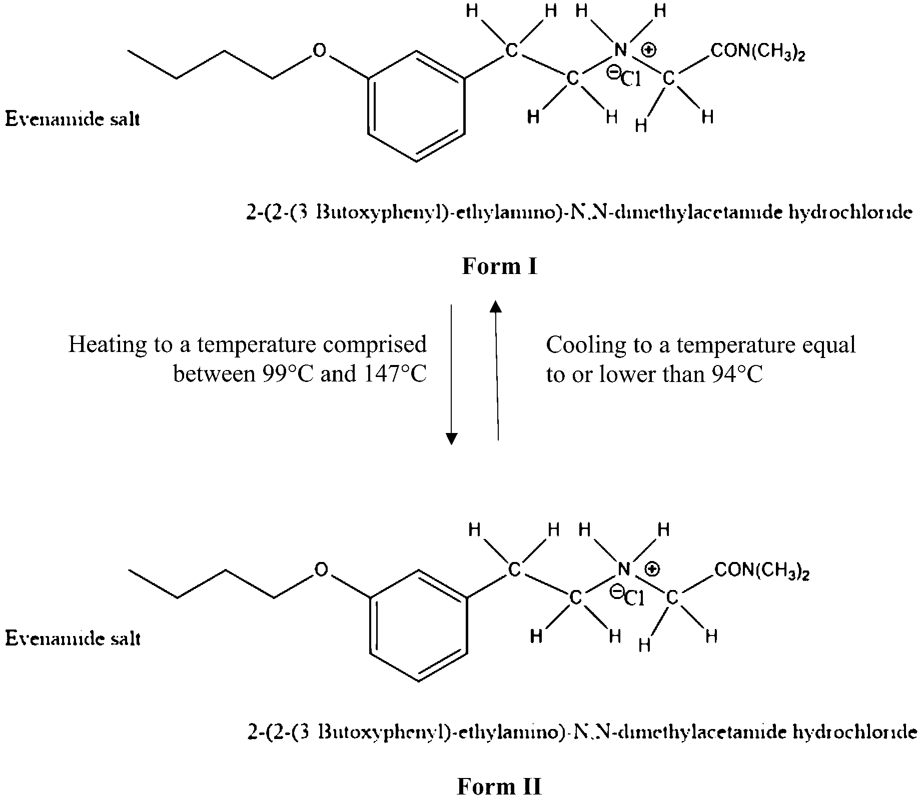

Evenamide in crystalline form I can be recovered by concentrating the resulting dispersion by methods known in the state of the art, for example by filtration and/or centrifugation.

Evenamide in crystalline form II can be recovered by concentrating the resulting dispersion by methods known in the state of the art, for example by filtration and/or centrifugation at a temperature higher than 94°C, preferably between 99 and 147°C.

All embodiments disclosed in the present invention may be combined each other.

Surprisingly, the inventors of the present application have found that evenamide in crystalline form I and II, obtained according to the invention keeps its crystalline form, even in the presence of moisture, as it can be appreciated for example by the XRPD spectra of Figure 5. This characteristic of evenamide in crystalline form of the invention makes it more stable than any other already known forms of evenamide and enables the storage, even for prolonged time periods, of the pharmaceutical formulations incorporating it as active pharmaceutical ingredient.

Evenamide in crystalline form I, as defined herein, is preferably an anhydrous crystalline form of evenamide.

Evenamide in crystalline form according to the invention is characterized by a high level of crystallinity. The term "high level of crystallinity" refers to a crystal form wherein the percentage of crystallinity is equal to or higher than 90%, preferably higher than 95% w/w as determined by standard methods of analysis, such as X-ray powder diffraction or microcalorimetry.

The chemical purity of evenamide in crystalline form according to the invention, evaluated by HPLC analysis as Area % (A%), is equal to or greater than 98%, and preferably equal to or greater than 99.5%.

A further object of the invention is a pharmaceutical composition comprising evenamide, preferably its hydrochloride salt, in crystalline form as above defined, preferably form I or II, and a pharmaceutically acceptable excipient and/or carrier.

Said pharmaceutical composition can be prepared in a pharmaceutical form by known methods. The dose of active ingredient presents in said composition may be that commonly used in clinical practice for evenamide.

A further object of the invention is evenamide, preferably its hydrochloride salt, in crystalline form as above defined, preferably form I and/or form II, for use as a medicament, preferably in the prevention and/or treatment of neurological, cognitive, psychiatric, inflammatory, urogenital and gastrointestinal diseases where a mechanism of action implying an aberrant sodium channel activity has been described as playing a pathological role, more preferably in the prevention and/or treatment of cognitive and/or psychiatric disorders such as schizophrenia.

A further object of invention is evenamide, preferably its hydrochloride salt, in crystalline form as above defined, preferably form I and/or form II, for use in the prevention and/or treatment of schizophrenia in add on, as a fixed or loose combination, to one or more, typical or atypical, antipsychotic agents, wherein the antipsychotic agent is preferably selected from the group comprising, or consisting of, risperidone, olanzapine, aripiprazole, clozapine, quetiapine, paliperidone, trifluoperazine, haloperidol, amisulpride, blonanserin; more preferably the typical antipsychotic agent is haloperidol, and the atypical antipsychotic agent is selected from risperidone and clozapine.

A further object of the invention is the use of evenamide, preferably its hydrochloride salt, in crystalline form as above defined, preferably form I and/or form II, to prepare a medicament for the prevention and/or treatment of cognitive and/or psychiatric disorders such as schizophrenia.

A further object of this invention is the use of evenamide, preferably its hydrochloride salt, in crystalline form as above defined, preferably form I and/or form II, to prepare a medicament for the prevention and/or treatment of schizophrenia in add on, as a fixed or loose combination, to typical or atypical antipsychotic agents, as defined above.

A further object of this invention is the use of evenamide, preferably its hydrochloride salt, in crystalline form as above defined, preferably form I and/or form II, to prepare a medicament for the prevention and/or treatment of schizophrenia in add on, as a fixed or loose combination, to typical or atypical antipsychotic agents, wherein the typical antipsychotic agent is haloperidol, and the atypical antipsychotic agent is selected from risperidone and clozapine.

A further object of the invention is a method for treating schizophrenia, comprising administering a therapeutically effective amount of evenamide, preferably its hydrochloride salt, in crystalline form as above defined, preferably form I and/or form II, to a patient in need thereof.

A further object of the invention is a method for treating schizophrenia, comprising administering a therapeutically effective amount of evenamide, preferably its hydrochloride salt, in crystalline form as above defined, preferably form I and/or form II, to a patient in need thereof in add on, as a fixed or loose combination, to typical or atypical antipsychotic agents, as defined above.

A further object of the invention is a method for treating schizophrenia, comprising administering a therapeutically effective amount of evenamide, preferably its hydrochloride salt, in crystalline form as above defined, preferably form I and/or form II, to a patient in need thereof in add on, as a fixed or loose combination, to typical or atypical antipsychotic agents, wherein the typical antipsychotic agent is haloperidol and the atypical antipsychotic agent is selected from risperidone and clozapine.

The following examples further illustrate the invention.

### Example 1 - Preparation of evenamide hydrochloride in crystalline form I

In a standard jacket reactor (1L) crude free base of evenamide 84.3 g, as a yellowish viscous oil, having a HPLC purity of 90% (Area%, A%), was added to acetone (320 ml) under mechanical stirring and nitrogen atmosphere, keeping the temperature at 25-30°C. HCl gas was then added below the mixture surface in the amount required for the stoichiometric salt formation. During the acid addition the temperature was increased by 5°C and the formation of a white to yellow precipitate was observed. The suspension was then cooled to 0°C and kept at this temperature for one additional hour. The suspension was filtered off and washed with acetone (2 x 40 ml). The white cake was then dried under vacuum at 30°C to provide evenamide HCl salt form I as a white needle solid (chromatographic purity 99%, A%).

### Example 2 - Preparation of evenamide hydrochloride in crystalline form I

In a glass reactor acetone (5.7 L) solution of evenamide free base (1.5 kg, purity 88% (A%), about 4.74 mol) was prepared and heated to 50°C and HCl gas (0.22 kg, 6.03 mol) was bubbled in about one hour.

The mixture was kept at 20°C for additional two hours and cooled down to 0°C in four hours. Finally, the mixture was kept at 0°C for three hours and filtered on a glass filter.

The white cake was then washed with acetone two times (2x 1.5 L) and dried at 40°C under vacuum to provide evenamide HCl salt form I (1.16 kg, purity 99.5 (A%), assay 99.5%, 3.50 mol, 74% molar yield).

### Example 3 - Recrystallization by solvent evaporation method of evenamide HCl salt form I

### 1. Recrystallization solvent: Methyl Ethyl Ketone

Evenamide HCl salt (650 mg) was dissolved in aqueous solution of H₂O (0.25 g) in methyl ethyl ketone (35 ml ), at room temperature. Evaporation of water and of methyl ethyl ketone, by storing the solution under vacuum, provided a residue consisting of evenamide HCl salt form I.

### 2. Recrystallization solvent: Methyl Isobutyl Ketone

Evenamide HCl salt (650 mg) was dissolved in methyl isobutyl ketone (15 ml), wet by H₂O (0.125 g), at room temperature. Evaporation of water and of methyl isobutyl ketone, under vacuum, provided a residue consisting of evenamide HCl salt form I.

### 3. Recrystallization solvent: Methyl Alcohol

Evenamide HCl salt (650 mg) was dissolved in methyl alcohol (5 ml), at room temperature. Evaporation was carried out under vacuum and provided a residue consisting of evenamide HCl salt form I.

### 4. Recrystallization solvent: Acetone

Evenamide HCl salt (650 mg) was dissolved in acetone (5 ml), at room temperature. Evaporation was carried out under vacuum and provided a residue consisting of evenamide HCl salt form I.

### Example 4 - Recrystallization by slurry method of evenamide HCl salt

### 1. Recrystallization solvent: Methyl Ethyl Ketone

A heterogeneous mixture of evenamide HCl salt (1.5 g) and methyl ethyl ketone (20 ml) was stirred in a round glass flask (50 ml) at 83°C for 12 h.

The warm suspension was then filtered on a glass filter. The solid was placed under vacuum at room temperature to remove the solvent providing 1.3 g of pure evenamide HCl salt form I. Yield 84% (solid recovered/solid initial amount).

### 2. Recrystallization solvent: Acetone

A heterogeneous mixture of evenamide HCl salt (1.5 g) and acetone (20 ml) was stirred in a round glass flask (50 ml) at 52°C for 12 h.

The warm suspension was then filtered on a glass filter. The solid was placed under vacuum at room temperature to remove the solvent providing 0.15 g of pure evenamide HCl salt form I.

### 3. Recrystallization solvent: Methyl Isobutyl Ketone

A heterogeneous mixture of evenamide HCl salt (1.5 g) and methyl isobutyl ketone (20 ml) was stirred in a round glass flask (50 ml) at 111°C (b.p. 116°C) for 12 h.

The warm suspension was then filtered on a glass filter. The solid was placed under vacuum at room temperature to remove the solvent providing 0.69 g of pure evenamide salt HCl form I.

### 4. Recrystallization solvent: Isopropyl Alcohol

A heterogeneous mixture of evenamide HCl salt (1.5 g) and isopropyl alcohol (20 ml) was stirred in a round glass flask (50 ml) at 77°C for 12 h.

The warm suspension was then filtered on a glass filter. The solid was placed under vacuum at room temperature to remove the solvent providing 0.66 g of pure evenamide HCl salt form I.

### Example 5 - XRPD measurements of a sample of Evenamide HCl

XRPD measurements of sample of evenamide HCl obtained according to Examples 1-4, after previous gentle grinding in an agata mortar, were taken in a TTK Anton Paar high temperature device using a Bruker D5005 diffractometer with CuKα, radiation, Ni filter, and position sensitive detector at the following conditions: 40kV, 30mA, stepwidth 0.015°(2θ), steptime 0.30 s/step, in angular range 4.5 <2θ <35.

Patterns were collected at 25°, 80° and 110°C, with a heating rate of 3°C/min and 20 min of delay time before each measurement.

At the end the sample was cooled to room temperature and a final pattern was collected at 25°. Then the sample was heated up to 110°C.

All the patterns at 110°C have shown to be of form II, but very different from those of form I collected at temperatures lower than 80°C.

The comparison indicates high degree of similarity of the crystalline phase of the samples, for both angular positions and intensity of the reflections for patterns collected in the temperature range 25-80°C. With increasing temperature, a slight angular decrease of the peaks is observed as expected for thermal expansion.

### Example 6 - Preparation of evenamide HCl salt in crystalline form III

### Slurry at RT

About 20 mg of Evenamide HCl salt as starting material was suspended in 0.2~0.5 mL of dioxane and dioxane water mixtures in HPLC vials. The suspension was then stirred magnetically for ~5 days at RT. Form III was observed from slurry in 1,4-dioxane (H₂O) solvent system. Form III sample was obtained via drying under N₂ at RT, and no form change was observed before/after drying, evidenced by XRPD (Figure 7).

The TGA result showed a weight loss of 3.7% from RT to 100 °C, and a weight loss of 7.7% from 100 °C to 160 °C.

The DSC result showed four endothermic peaks at 56.6 °C, 88.0 °C, 98.6 °C and 149.4 °C (peak).

¹H NMR spectrum showed the molar ratio of 1,4-dioxane/Evenamide HCl was 0.5 (12.3 wt%).

Form III was further heated to study the thermal events, and the XRPD result showed Form I was formed after heating to 70 °C. The PLM image showed Form III consisted of plate-like crystals and agglomerates.

The remaining solids were isolated before XRPD analysis. The results are reported in Table 5.

**Table 5 - Summary of slurry experiments at RT**

| **Solvent (v/v)** | **Results** |
|---|---|
| 1,4-Dioxane | Form I+III |
| 1,4-Dioxane/H₂O (98:2, a_{w}~0.2) | Form III |
| 1,4-Dioxane/H₂O (95:5, a_{w}~0.4) | Form III |
| 1,4-Dioxane/H₂O (91:9, a_{w}~0.6) | Form III |
| 1,4-Dioxane/H₂O (84:16, a_{w}~0.8) | Form III |

Based on the characterization results, Form III was determined to be a 1,4-dioxane solvate of mono-HCl salt.

### Example 7 - Preparation of evenamide mucate ((2S,3R,4S,5R)-2,3,4,5-tetrahydroxyhexanedioic acid salt of evenamide)

Evenamide mucate was prepared by reactive crystallization. Mucic acid *((2S,3R,4S,5R)-*2,3,4,5-tetrahydroxyhexanedioic acid) was added to a 50 mg/ml solution of evenamide free base in methyl-ethyl ketone in a molar ratio of 1:1.

The mixture was agitated for 3 days at room temperature and the solid isolated by centrifugation. The solids were then dried under nitrogen. Polarized light microscopy showed the solid to be bi-refringent crystals.

TGA/DSC indicated negligible weight loss to 150 °C, and one endothermic peak at 154.8 °C (melt). The ¹H NMR result was obtained in DMSO-d6 and confirmed that the stoichiometry of acid/base was 1.0, with no residual solvent. The purity by HPLC was 99.6 area%. Based on the characterization data, mucate was assessed to be anhydrate of mono salt. The crystallinity was confirmed and characterized by XRPD.

### Example 8 - Preparation of evenamide citrate 2-hydroxypropane-1,2,3-tricarboxylic acid salt of evenamide

Evenamide citrate was prepared by reactive crystallization. citric acid (2-hydroxypropane-1,2,3-tricarboxylic acid) was added to a 50 mg/ml solution of evenamide free base in 2 methyl tetrahydrofuran in a molar ratio of 1:1. The mixture was agitated for 5 days at room temperature and the solid isolated by centrifugation. The solids were then dried under Nitrogen. PLM showed the solid to be bi-refringent crystals.

TGA/DSC indicated a weight loss of 0.22% to 120 °C, and one endothermic peak at 140.7 °C (melt). The ¹H NMR result was obtained in DMSO-d6 and confirmed that the stoichiometry of acid/base was 1.1, with no residual solvent. HPLC showed the purity was 100.0 area%. Based on the characterization data, citrate was assessed to be anhydrate of mono salt. The crystallinity was confirmed by XRPD.

### Example 9 - Preparation of evenamide hemi-edsylate (1,2 ethane disulfonic acid salt)

Edisylate Type A was prepared by reactive crystallization. 1,2 ethane di-sulfonic acid was added to a 50mg/ml solution of evenamide free base in isopropanol/n-heptane (1:5, v/v) in a molar acid base ratio of 0.5:1. The mixture was agitated at RT for 3 days followed by agitation at 5 °C overnight. The solid product was isolated by centrifugation and dried under nitrogen. PLM image of the solid showed birefringent crystals.

TGA/DSC indicated a 0.15% weight loss to 120 °C. The DSC indicated three endothermic peaks at 115.0 °C, 148.0 °C and 197.8 °C. The stoichiometry of acid/base was 0.5 as determined by 1H NMR in DMSO-d6. The purity by HPLC s was 99.3 area%.

A heating experiment was conducted on edisylate Type A. After heating to 120 °C, edisylate Type A changed to edisylate Type B. Based on the characterization data, edisylate Type A was likely an anhydrate of hemi salt, and Edisylate type B was a different crystalline form.

Edisylate Type B was prepared directly by reactive crystallization in 2-Me-THF. 1,2 ethane disulfonic acid was added to a 50mg/ml solution of evenamide free base in 2-methyl tetrahydrofuran at a molar ratio of 0.5 (acid/base). The mixture was agitated at RT for 3 days followed by agitation at 5 °C overnight. The solid product was isolated by centrifugation and dried under nitrogen PLM image showed birefringent crystals.

TGA/DSC showed a 0.21% weight loss by 120 °C, and two endothermic peaks at 148.3 °C and 200.3 °C (peak temperature). The ¹H NMR result was obtained in DMSO-d6 and showed that the stoichiometry of acid/base to be 0.5. The purity by HPLC was 98.7 area%.

The resulting edisylate Type B was characterized by VT-XRPD. After heating to 150 °C, edisylate Type B changed to a new crystal form, named as edisylate Type C, and converted back to edisylate Type B upon return to RT. Based on VT-XRPD and characterization data, edisylate Type B/C were likely enantiotropically related anhydrate of hemi salts. The XRPD indicated a different crystalline structure for Type A and Type B.

### Example 10 - Preparation of evenamide mesylate

The mesylate salt was prepared by reactive crystallization. A 50 mg/ml solution of evenamide free base in ethyl acetate was reacted with methane sulfonic acid in a molar ratio of 1:1. The mixture was agitated at RT for 3 days followed by agitation at 5 C overnight. The solids were isolated by centrifugation and dried under nitrogen. PLM image showed plate-like birefringent crystals.

TGA/DSC indicated negligible weight loss to 150 °C, and one endothermic peak at 139.5 °C (peak temperature). The 1H NMR result was obtained in DMSO-d6 and indicated that the stoichiometry of acid/base was 0.9, with no residual solvent.

HPLC showed the purity was 100.0 area%. Based on the characterization data, mesylate Type A was determined to be the anhydrate of the mono salt.

### Example 11 - Preparation of evenamide napsylate

The napsylate salt was prepared in by reactive crystallization at RT. A 50 mg/ml solution of evenamide free base in 2-methyl tetrahydrofuran was reacted with methane sulfonic acid at RT for 3 days followed by cooling to 5 C overnight. The solids were isolated by centrifugation and dried under nitrogen. The PLM image showed birefringent crystals.

TGA/DSC indicated 0.05% weight loss to 130 °C, and one endothermic peak at 184.5 °C (peak temperature). The ¹H NMR result obtained in DMSO-d6 and indicated that the stoichiometry of acid/base was 0.9, with no residual solvent.

HPLC showed the purity was 98.8 area%. The water content was 0.02% determined by KF. Based on the characterization data, napsylate Type A was an anhydrate of mono salt.

### Example 12 - Preparation of evenamide saccharinate (1H-1λ⁶2-Benzothiazole-1,1,3(2H)-trione salt)

The saccharinate salt was prepared by reactive crystallization. A 50 mg/ml solution of evenamide free base in isopropanol/n-heptane (1:5, v/v) was agitated with 1*H*-1λ⁶,2-Benzothiazole-1,1,3(2*H*)-trione in a molar ratio of 1:1 at RT for 3 days. The resulting solid was isolated by centrifugation and dried under nitrogen. PLM image showed birefringent crystals.

TGA/DSC indicated negligible weight loss to150 °C, and one endothermic peak at 150.1 °C (peak temperature). The ¹H NMR result was obtained in DMSO-*d6* and indicated that the stoichiometry of acid/base was 0.9, with no residual solvent.

The purity by HPLC was 100.0 area%. Based on the characterization data, saccharinate was determined to be an anhydrate of mono salt.

### Conclusive Remarks

Evenamide hydrochloride has been found to exist in at least three enantiotropic polymorphs, namely form I, stable at room temperature, form II stable above the transition temperature (94°C to 99°C) and form III, a 1,4-dioxane solvate, stable at room temperature.

Form I was observed (DSC and XRPD) to convert reversibly into Form II when heated above 99°C.

It is worth noting that DSC and XRPD diagrams have been registered on solid product in the absence of solvent.

## Claims

1. A crystalline form of evenamide salt designated as form I, having an XRPD spectrum with the characteristic peaks, measured using Cu Kα radiation (λ = 1.54 Å), falling at about 12.1; 13.4; 16.2; 16.7; 18.2; 18.7; 19.4; 21.7; 22.4; 22.8; 23.3; 23.7; 24.2; 24.4; 25.4; 26.0; 26.6 ± 0.2° in 2θ.

2. A crystalline form of evenamide salt designated as form II, having an XRPD spectrum with the characteristic peaks, measured using Cu Kα radiation (λ = 1.54 Å), falling at about 12.7; 17.7; 20.0; 20.4; 20.7 ± 0.2° in 2θ.

3. A crystalline form of evenamide salt according to claim 1 or 2, wherein the salt is hydrochloride.

4. A crystalline form of evenamide salt which is selected from a salt exhibiting a predominant endothermic peak:
- at about 119°C, preferably sulfate;
- at about 132.6°C, preferably maleate;
- at about 105.6°C, preferably phosphate;
- at about 118.3°C, preferably pyruvate;
- at about 114.6°C, preferably fumarate;
- at about 154.8°C, preferably mucate;
- at about 140.7°C, preferably citrate;
- at about 101.2°C, preferably L-malate;
- at about 85.8°C, preferably succinate;
- at about 91.4°C, preferably glutarate;
- at about 147.9°C, preferably napadisylate;
- at about 197.8°C, preferably edisylate;
- at about 120.0°C, preferably esylate;
- at about 161.8°C, preferably tosylate;
- at about 139.5°C, preferably mesylate;
- at about 184.5°C, preferably napsylate;
- at about 107.0°C, preferably besilate;
- at about 198.3°C, preferably oxalate;
- at about 96.6°C, preferably malonate;
- at about 150.1°C, preferably saccharinate;
- at about 129.0°C, preferably camsylate; and
- at about 105.0°C, preferably mandelate.

5. A crystalline form of evenamide salt according to claim 4, wherein the salt is selected from maleate, mucate, citrate, edisylate, mesylate, napsylate, oxalate, saccharinate and camsylate.

6. A crystalline form of evenamide salt according to claim 5, wherein the salt is selected from mucate, citrate, edisylate, mesylate, napsylate and saccharinate.

7. A process for the preparation of evenamide in crystalline form I, as defined in claim 1, comprising:
• adding a solvent to evenamide free base and hydrochloric acid in gas form, or aqueous hydrochloric acid, preferably hydrochloric acid in gas form, as a salification reagent);
• crystallizing at a temperature equal to or lower than 94°C;
• recovering evenamide hydrochloride salt in crystalline form I;
wherein the solvent is selected from acetone, water, methyl alcohol, isopropyl alcohol, methyl ethyl ketone/water, ethyl acetate, methyl isobutyl ketone, methyl isobutyl carbinol, dimethyl formamide, heptane(s), or a mixture of said solvents, preferably the solvent is acetone.

8. A process for the preparation of evenamide hydrochloride salt in crystalline form II according to claim 2, comprising:
• adding a solvent to evenamide free base;
• heating the solution/dispersion to a temperature comprised between 99 and 147°C;
• crystallizing by adding a salification reagent;
• obtaining and optionally recovering evenamide hydrochloride salt in crystalline form II.

9. A process for the preparation of evenamide hydrochloride salt in crystalline form II according to claim 2 or 3, comprising:
• adding a solvent to evenamide free base and a salification reagent to form evenamide hydrochloride salt in crystalline form I;
• heating the solution/dispersion to a temperature comprised between 99 and 147°C;
• obtaining and optionally recovering evenamide hydrochloride salt in crystalline form II.

10. A process for the preparation of evenamide hydrochloride salt in crystalline form I according to claim 1, comprising:
• adding a solvent to evenamide free base;
• heating the solution/dispersion to a temperature comprised between 99 and 147°C;
• crystallizing by adding hydrochloric acid in gas form as salification reagent;
• obtaining evenamide hydrochloride salt in crystalline form II,
• cooling down to a temperature comprised between 0 and 94°C, preferably at a temperature comprised between 20 and 30°C, more preferably of 25°C (room temperature);
• obtaining and recovering evenamide hydrochloride salt in crystalline form I.

11. A process for the preparation of evenamide hydrochloride salt in crystalline form I according to claim 1, comprising:
• adding a solvent to evenamide free base and hydrochloric acid in gas form as salification reagent;
• heating the solution/dispersion to a temperature comprised between 99 and 147°C;
• obtaining evenamide hydrochloride salt in crystalline form II,
• cooling down to a temperature comprised between 0 and 94°C, preferably at a temperature comprised between 20 and 30°C, more preferably of 25°C (room temperature);
• obtaining and recovering evenamide hydrochloride salt in crystalline form I.

12. A process according to claims 4-7, wherein the solvent is selected from acetone, water, methyl alcohol, isopropyl alcohol, methyl ethyl ketone/water, ethyl acetate, methyl isobutyl ketone, dimethyl formamide, heptane(s), or a mixture of said solvents, preferably the solvent is acetone.

13. A process according to claims 4-8, wherein the salification reagent is selected from hydrochloric acid in gas form, and aqueous hydrochloric acid; preferably hydrochloric acid in gas form.

14. Use of crystalline form I of evenamide as defined in claim 1 or 3, in the preparation of evenamide in crystalline form II as defined in claim 2 or 3, by heating crystalline form I of evenamide at a temperature comprised between 99°C and 147°C to obtain evenamide in crystalline form II.

15. Use of crystalline form II of evenamide as defined in claim 2 or 3, in the preparation of evenamide in crystalline form I as defined in claim 1 or 3, by cooling crystalline form II of evenamide at a temperature equal to or lower than 94°C to obtain evenamide in crystalline form I.

16. A pharmaceutical composition comprising a salt of evenamide in crystalline form according to claims 1-6, as active pharmaceutical ingredient, and a pharmaceutically acceptable excipient and/or carrier.

17. Evenamide salt in crystalline form according to claims 1-6, for use as a medicament.

18. Evenamide salt in crystalline form according to claims 1-6, for use in the prevention and/ or treatment of neurological, cognitive, psychiatric, inflammatory, urogenital and gastrointestinal diseases, wherein an aberrant sodium channel activity plays a pathological role.

19. Evenamide salt in crystalline form according to claims 1-6, for use according to claim 18, wherein the disease is a psychiatric disorder such as schizophrenia.

20. Evenamide salt in crystalline form according to claims 1-6, for use according to claim 19 in add on to one or more antipsychotic agents.

21. Evenamide salt in crystalline form according to claims 1-6, for use according to claim 20, wherein the antipsychotic agent is selected from the group comprising, or consisting of, risperidone, olanzapine, aripiprazole, clozapine, quetiapine, paliperidone, trifluoperazine, haloperidol, amisulpride and blonanserin.

22. Evenamide salt in crystalline form according to claims 1-6, for use according to claim 20 in add on to one or more typical or atypical antipsychotic agents, wherein the typical antipsychotic agent is haloperidol and the atypical antipsychotic agent is selected from risperidone and clozapine.
